(19) **Europäisches Patentamt / European Patent Office / Office européen des brevets**

(11) **EP 2 294 932 B1**

(12) **EUROPEAN PATENT SPECIFICATION**

(45) Date of publication and mention of the grant of the patent:
**21.10.2015 Bulletin 2015/43**

(21) Application number: **08761564.7**

(22) Date of filing: **07.05.2008**

(51) Int Cl.:
*A23L 1/29* (2006.01)     *A61K 31/718* (2006.01)
*A61P 3/08* (2006.01)     *A61P 3/02* (2006.01)

(86) International application number:
**PCT/ES2008/000319**

(87) International publication number:
**WO 2009/135960 (12.11.2009 Gazette 2009/46)**

(54) **CARBOHYDRATE MIXTURE AND THE USE THEREOF FOR PREPARING A PRODUCT FOR ORAL OR ENTERAL NUTRITION**

KOHLENHYDRATMISCHUNG UND IHRE VERWENDUNG FÜR DIE HERSTELLUNG EINES PRODUKTS FÜR DIE ORALE ODER ENTERALE ERNÄHRUNG

MÉLANGE D'HYDRATES DE CARBONE ET SON UTILISATION POUR PRÉPARER UN PRODUIT DESTINÉ À L'ALIMENTATION PAR VOIE ORALE OU ENTÉRALE

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**16.03.2011 Bulletin 2011/11**

(73) Proprietor: **Vegenat, S.A.**
**06184 Pueblonuevo del Guadiana, Badajoz (ES)**

(72) Inventors:
• **GIL HERNANDEZ, Ángel**
  **E-06011 Badajoz (ES)**
• **SAN ROMÁN PAIS, Paloma**
  **E-06011 Badajoz (ES)**

• **PEREZ RODRIGUEZ, Milagros**
  **E-06011 Badajoz (ES)**

(74) Representative: **Gil-Vega, Victor**
  **Corazón de Maria, 6**
  **28002 Madrid (ES)**

(56) References cited:
**EP-A1- 0 756 828      EP-B1- 0 768 043**
**WO-A1-2004/022074      WO-A1-2005/036971**
**WO-A2-2006/108008      WO-A2-2007/004883**
**WO-A2-2007/070616**

Remarks:
The file contains technical information submitted after the application was filed and not included in this specification

**Description**

The present invention refers to a new mixture of carbohydrates and its use in the preparation of a food product intended for oral or enteral nutrition, especially for the nutrition of the diabetic patient and in particular for the nutrition of the diabetes type II patient.

Whether the form of administration be oral or enteral, a large number of social groups exist which need their food requirements to be covered by this type of product. With regard to the subject under discussion, a particularly important group is that formed by mellitus diabetes patients, especially non insulin dependent mellitus diabetes or diabetes type II.

Diabetes mellitus type 2 accounts for 80-90% of diabetes cases. The frequency of diabetes mellitus is rapidly increasing on a worldwide scale. In 1997 data regarding prevalence was around 124 million people; it is estimated that in the year 2025 this figure will have increased to 300 million. The growing proportion of diabetes type II and the fact that disorders relating to insulin resistance and its metabolic complications contribute to cardiovascular disease (Mann 2000) have increased the interest in the nutritional determinants of these metabolic complications. In this type of diabetes diverse metabolic disorders take place which are characterised by an inappropriate rise in blood glucose (hyperglycemia), which leads to chronic complications through transformation of large and small vessels and nerves. The underlying alteration in this disease is the difficulty for the insulin to act (as a loss of sensitivity of the tissues to this hormone) called "insulin resistance" and an insufficient production of insulin by the cells responsible for its production in the pancreas. As well as increasing the concentration of glucose, the defective action of the insulin frequently includes a rise in the levels of cholesterol and/or triglycerides. Most cases of diabetes mellitus type 2 occur within the context of the so-called "metabolic syndrome", to which diabetes, high blood pressure, an increase in cholesterol, triglycerides and/or uric acid levels and overweight are associated. The metabolic syndrome notably raises cardiovascular risk and is a serious cause of death in developed countries.

The treatment of diabetes type 2 and its associated comorbidity is based upon three essential pillars: following a balanced diet plan, getting regular physical exercise and personalised pharmacological treatment.

The American Association of Diabetes and the European Association for the Study of Diabetes make a general recommendation regarding the carbohydrates present in the diet of a diabetic person: they recommend low-glycemic index foods and foods rich in soluble fibres, as well as a saccharose intake lower than 10% of the acceptable total energy in certain circumstances (FAO Food and nutrition study, 1997).

Apart from being the main substrata of energetic metabolism, carbohydrates can affect satiety, blood glucose, insulin levels, lipid metabolism and, through fermentation, exercise a major control in the functioning of the colon, including the passage through the intestines, the metabolism and balance of intestinal flora and the cellular state of the epithelium of the large intestines. They may also act as immunomodulators and influence the absorption of calcium. These properties have implications in general health, contributing, in particular, to body weight control, diabetes, cardiovascular disease, bone density, large intestine cancer, constipation and intestinal resistance to infections (Cummings, J.H., Carbohydrate: Terminology and Classification, European Journal of Clinical Nutrition, 2007).

Several controlled trials revealed that diets with a high content of soluble fibre or low-glycemic index foods are associated with an improvement in the daily glycemic profiles, and a long term improvement of glycemic control, as proven by a lower level of glycated haemoglobin (Mann, J.I., Lines to legumes: changing concepts of diabetic diets; Diabetic Medicine 1984, 1:191-198; Brand, J.C. Colagiuri, S., Crossman, S., Allen, A. Roberts, D.C., Truswell, A.S., Low-glycemic index foods improve long-term glycemic control in NIDDM; Diabetes Care 1991, 14:95-101).

In this respect, the glycemic index (GI) is a universally accepted index which is used to classify foods based on their potential to increase glycemia. The glycemic index is defined as the area of increase under the curve of glycemic response (AUC) of a portion of 50 g of carbohydrates of a trial food, expressed in percentage of response to the same amount of carbohydrate of a standard foodstuff (white bread or glucose) ingested by the same subject (Consult FAO/WHO of experts on carbohydrates in human nutrition, 1997). Monosaccharide glucose induces a high glycemic response and is the most frequently used model, being given a GI value of 100. This index has been extended to take into account the effect of the total amount of carbohydrates consumed, and, thus, the glycemic load (GL) is defined as the product of the glycemic index (GI) and the amount of carbohydrates ingested, and provides an indication of the glucose available as energy and for storage after ingestion (postprandial levels).

According to the FAO/WHO Expert Consultation on Carbohydrates in Human Nutrition (1997), foods with a low GI reduce both postprandial glycemia and insulin response. Trials with animals suggest that the incorporation of slow digestion starch into the diet delays the start of insulin resistance. Several epidemiological trials indicate that a diet with low GI is associated with a reduction in the risk of suffering from non insulin-dependent diabetes in the man and the woman. Clinical trials in normal, diabetic and hyperlipidemic subjects prove that the diets with low GI reduce average glucose concentrations levels, reduce insulin secretion and reduce the seric triglycerides in individuals with hypertriglyceridemia. Furthermore, the digestibility of carbohydrates in low GI foods is generally lower than that of foods with high GI. For this reason, foods with low GI increase the amount of carbohydrates entering into the colon and increase fermentation and the production of short chain fatty acids, which has implications for body nitrogen, lipidic metabolism and local phenomena in the colon.

There are several types of formulations on the market for oral or enteral nutrition which have been especially designed for the diabetic patient, formed by a mixture of specific nutrients and classified as "dietary products for specific nutritional uses" within the legal framework. In general, they consist of complete formulas, containing the appropriate quantity of each and every nutrient necessary for maintaining a balanced nutritional state, in supplements, which are incomplete and complement an appropriate oral diet, and, lastly, in modules which contain a specific nutrient. In the case of oral administration formulations other substances are added to improve palatability, for example flavours and aromatizers. Complete formulation products are of particular importance, where the nutritive formula contains proteins, carbohydrates, fats, fibre and micronutrients, as the T-Diet® product range by the same applicant does.

Among the said commercial products the following may be mentioned, for example: Nutrison®Diabetes (Nutricia), an enteral composition which contains a mixture of carbohydrates based on starch as the main carbohydrate and fructose (45% of the energy), as well as soluble and insoluble fibre, a high content of monounsaturated fatty acids, soya proteins and micronutrients. Diasip®, also by Nutricia, a composition for oral nutrition, contains a mixture of carbohydrates (35% of the energy) consisting of starch (6.8 g/100ml), fructose (1.9 g/100ml), saccharose (0.1 g/100ml) and lactose (< 0.025 g/100ml), as well as proteins, lipids, fibre and other micronutrients; Glucerna®Select, by Abbot, a complete product for enteral or oral nutrition, containing a mixture of carbohydrates (31% of the energy) consisting of maltodextrine, modified maltodextrine, fructose, maltitol, FOS and soya polysaccharides, as well as fibre; or the Novartis Medical Nutrition range of products for the diabetic or hyperglycaemic patient, which contain carbohydrates (starch and fructose), soluble fibre, proteins and lipids.

All the previously mentioned products contain a mixture of carbohydrates the total calorific provision of which is that recommended for diabetic patients, 45-65% of the energy according to the American Diabetes Association (2000/2006) and 45-60% of the energy according to the European Association for the Study of Diabetes (1999). Fructose is included in all of them to meet with the said recommendations.

The WO2004/022074, "Nutritional compositions comprising a non-glucose carbohydrate or pectin and soluble fibre", reveals a dietary composition which comprises carbohydrates except glucose, for example galactose, xylose, fructose or mannose, preferably galactose, in combination with soluble fibres for increasing the plasmatic levels of GLP-1 in plasma. The WO2007/004883, "A carbohydrate fraction and use thereof for a flat postprandial glucose response", reveals a nutritional product **characterised in that** the carbohydrate fraction comprises: a) 5-30% in weight of one or more selected monosaccharides from between galactose, ribose and manose; b) 5-65% in weight of carbohydrates capable of providing a delayed glucose release; c) optionally fructose; d) optionally disaccharide containing glucose other than maltose and saccharose and e) optionally poliols. The ES 2211932T3, "Nutritional product for diabetics with controlled carbohydrate absorption", is related to a nutritional composition comprising a carbohydrate component with a combination of ingredients which provide fast, moderate and show absorption of carbohydrate subsequent to consumption, resulting in a sustained carbohydrate release without excessive peaks of blood glucose, the carbohydrate component comprising between 1 and 90% of the total caloric load, wherein the said carbohydrate component comprises a quickly absorbed fraction comprising glucose, one or more disaccharides quickly absorbed which contain a unit of glucose or a mixture of the same, in which the said fraction includes saccharose; a fraction moderately absorbed which comprises one or more non glucose monosaccharides moderately absorbed, disaccharides which contain no glucose, polysaccharides which contain glucose, or mixtures of the same; and a slowly absorbed fraction which comprises one or more slowly absorbed polysaccharides which contain glucose.

Recent trials undertaken with animal models, see for example "Metabolic effects of fructose" (Lê, K-A. and Tappy, L., Current opinion in Clinical Nutrition and Metabolic Care 2006, 9: 469-475), have showed that fructose stimulates the lipogenesis and leads to hepatic and extra hepatic insulin resistance, dyslipidemia and arterial hypertension. The insulin-resistance seems to be related to ectopic fat depots. In humans, short term feeding with fructose increases the de novo lipogenesis and the blood triglyceride levels and provokes hepatic insulin resistance. The cellular mechanisms underlying the metabolic effects of fructose involve the production of reactive oxygen species, the activation of cellular stress routes and possibly an increase in uric acid synthesis. In human beings, the consumption of fructose may increase the concentrations of triglycerides in plasma and disturbs the hepatic homeostasis of glucose, although it does not appear to cause insulin-resistance in muscle nor increase blood pressure short term. John Bantle, M.D., University of Minnesota ("Effects of dietary fructose on plasma lipids in healthy subjects", John P. Bantle, Susan K. Raatz, William Thomas and Angeliki Georgopoulos, American Journal of Clinical Nutrition, Vol. 72, N° 5, 1128-1134, November 2000) carried out a trial in which two exactly the same diets were administered except that one of them contained fructose instead of starch to subjects who suffered from diabetes type I and type II. In both groups a reduction in blood glucose was confirmed. However, according to the Bantle report of November 1992 in "Diabetes Care", total cholesterol of the subjects was raised on average by 7% and their LDL rose by almost 11 %. Bantle noticed the same effects in a trial with healthy subjects. In this case, total cholesterol rose by approximately 9% and the LDL fraction by approximately 11%. Concluding that, bearing in mind the possible risk factors which these findings could involve, diets which contain fructose would not be the most appropriate for this type of patients.

The mixture of carbohydrates of this invention, apart from being new, successfully reduces the postprandial glycemic response, which is why it is useful for nutritional treatment or for the prevention of diseases associated with the metabolic syndrome in general and diabetes in particular, primordially that of diabetes type II, and its associated comorbidity, reducing the average concentrations of blood glucose, the secretion of insulin and seric triglycerides. Equally, the mixture of carbohydrates of the present invention, owing to their low glycemic index, increase the quantity of carbohydrate entering the colon and increase the fermentation and production of absorbable short chain fatty acids (SCFA), making possible the potential regulation of hepatic glyconeogenesis and insulin control, with obvious effects on the metabolism of the lipoproteins.

Therefore, the object of the present invention is to provide a mixture of carbohydrates for the nutritional treatment or for the prevention of diseases associated with the metabolic syndrome in general and diabetes in particular, primarily diabetes type II and its associated comorbidity, which will result in a reduction of the postprandial glycemic response, and which is free of fructose.

The mixture of carbohydrates of the present invention is composed by type IV or RS IV resistant starch (modified maltodextrine), maltodextrine of low dextrose equivalent DE = 5-8, preferably DE equal to 6, inulin (fructooligosaccharide) and cellulose. The proportion of each one of these components present in the mixture of the invention is as follows:

Type IV resistant starch: 50.98%, soluble fibre;
maltodextrine (5-8 DE): 33.74%;
inulin: 11.74%, soluble fibre, pre-biotic carbohydrate,
Cellulose: 3.54%, insoluble fibre.

The total proportion of sugars present in the mixture of carbohydrates of the invention is approximately that of 0.91%, where the said sugars come from the maltodextrine DE 5-8 (67%) and type IV resistant starch (33%).

In one form of embodiment of the invention, the mixture of carbohydrates of the present invention additionally includes a sweetener additive, preferably potasic acesulfame and/or a stabilizer, preferably carragenate I. The proportion of these additives present in the mixture is preferably that of 0.32% for the sweetener and 0.22% for the stabilizer, percentages in weight respect to the total weight of the mixture.

The modified maltodextrine or type IV resistant starch, comprising 50.98% of the carbohydrates present in the mixture of the invention, is incorporated into the mixture of the carbohydrates of the invention in the form of the commercial product Nutriose® FM06, by the company Roquette.

Description of the figures:

Figure 1: curve relating to the glycemic index obtained with the mixture of carbohydrates of the invention in comparison with a glucose standard in accordance with one example of embodiment.

Figure 2: curve relating to the insulin response obtained with the mixture of carbohydrates of the invention in comparison with a glucose standard in accordance with one example of embodiment.

Figure 3: curve relating to the C-peptide obtained with the mixture of carbohydrates of the invention in comparison with a glucose standard in accordance with one example of embodiment.

Figure 4: curve relating to the triglyceride level obtained with the mixture of carbohydrates of the invention in comparison with a glucose standard in accordance with one example of embodiment.

In an embodiment example, the mixture of carbohydrates of the invention is made up of the ingredients and quantities of the same shown in the following Table 1:

**Table 1**

| Ingredients | Quantity (g) | Proteins (g) | HC+ fibre (g) | Sugars (g) | Lipids (g) |
|---|---|---|---|---|---|
| Nutriose FM06 | 4,91 | 0,01473 | 4,17 | 0,02455 | 0,00491 |
| Maltodextrine 5-8 DE | 2,90 | - | 2,76 | 0,05 | - |
| Inulin | 0,99 | - | 0,96 | 0,00 | - |
| Cellulose | 0,30 | - | 0,29 | - | - |

(continued)

| Ingredients | Quantity (g) | Proteins (g) | HC+ fibre (g) | Sugars (g) | Lipids (g) |
|---|---|---|---|---|---|
| Ace-K | 0,03 | - | - | - | - |
| Carragenate I | 0,02 | - | - | - | - |
| Total | 9,15 | 0,01473 | 8,18 | 0,07455 | 0,00491 |

In this embodiment example, the amounts indicated correspond with the percentages of ingredients present in the mixture indicated in Table 2:

**Table 2**

| Ingredients | % |
|---|---|
| Nutriose FM06 | 53,66 |
| Maltodextrine 5-8 DE | 31,70 |
| Inulin | 10,81 |
| Cellulose | 3,28 |
| Ace-K | 0,33 |
| Carragenate I | 0,22 |
| Total | 100 |

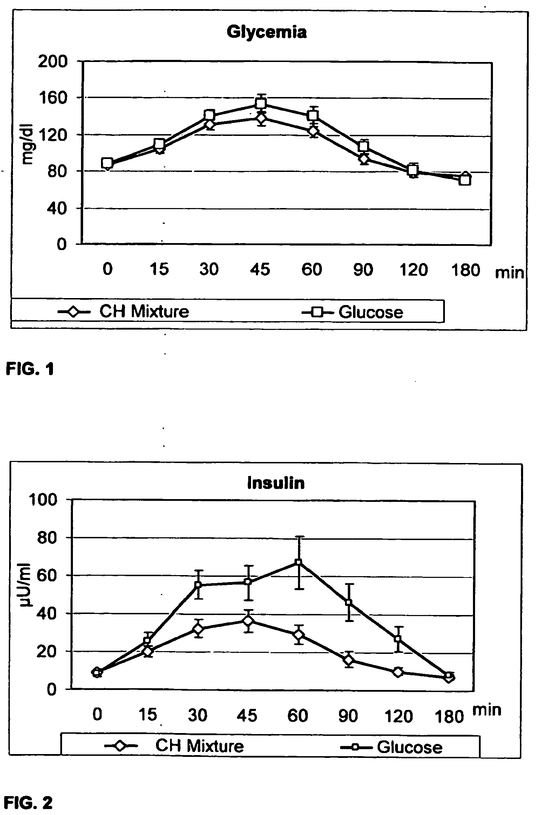

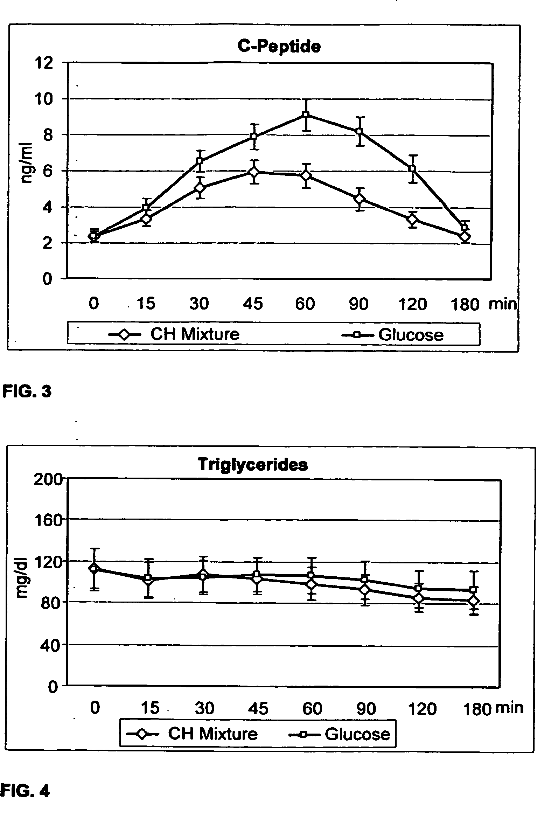

A trial was made with healthy volunteers to which the mixture of carbohydrates as of the present invention was administered orally. The findings obtained are revealed below (Figures 1 to 4):

| | | CH Mixture as of the invention | | 50 g of Glucose | |
|---|---|---|---|---|---|
| | Time (min) | Mean | SEM | Mean | SEM |
| Glucose | 0 | 87,5 | 2,4 | 88,1 | 3,4 |
| | 15 | 104,5 | 4,2 | 109,8 | 5,8 |
| | 30 | 131,5 | 5,5 | 140,4 | 7,6 |
| | 45 | 138,0 | 7,5 | 154,0 | 9,7 |
| | 60 | 125,0 | 7,9 | 140,6 | 11,0 |
| | 90 | 94,1 | 6,3 | 107,1 | 8,2 |
| | 120 | 78,7 | 2,0 | 81,9 | 7,6 |
| | 180 | 75,6 | 2,0 | 70,4 | 1,9 |
| Insulin | 0 | 9,2 | 1,4 | 8,2 | 1,6 |
| | 15 | 20,0 | 2,8 | 25,4 | 4,5 |
| | 30 | 32,4 | 4,7 | 55,3 | 7,7 |
| | 45 | 36,4 | 6,0 | 56,4 | 9,3 |
| | 60 | 29,4 | 5,2 | 67,3 | 13,7 |
| | 90 | 16,3 | 4,0 | 46,4 | 9,6 |
| | 120 | 10,3 | 2,0 | 27,1 | 6,7 |
| | 180 | 7,4 | 1,3 | 8,4 | 1,7 |
| C-Peptide | 0 | 2,4 | 0,4 | 2,4 | 0,3 |
| | 15 | 3,4 | 0,4 | 3,9 | 0,5 |
| | 30 | 5,1 | 0,6 | 6,5 | 0,6 |
| | 45 | 5,9 | 0,7 | 7,9 | 0,7 |
| | 60 | 5,7 | 0,7 | 9,1 | 0,9 |
| | 90 | 4,5 | 0,6 | 8,2 | 0,8 |
| | 120 | 3,3 | 0,5 | 6,1 | 0,8 |

(continued)

| | Time (min) | CH Mixture as of the invention | | 50 g of Glucose | |
|---|---|---|---|---|---|
| | | Mean | SEM | Mean | SEM |
| | 180 | 2,4 | 0,4 | 2,9 | 0,4 |
| TAG | 0 | 112,5 | 19,6 | 111,6 | 20,6 |
| | 15 | 101,2 | 17,1 | 103,3 | 18,4 |
| | 30 | 107,4 | 17,2 | 104,8 | 16,4 |
| | 45 | 104,0 | 16,0 | 107,5 | 16,0 |
| | 60 | 98,8 | 15,9 | 106,8 | 17,4 |
| | 90 | 92,9 | 14,8 | 102,8 | 18,3 |
| | 120 | 85,5 | 13,6 | 94,0 | 18,1 |
| | 180 | 83,3 | 12,9 | 93,1 | 18,3 |
| TAG = Triacilglycerides | | | | | |

The findings for the area under the glycemic response curve obtained in the subjects of the trial are contained in the following Table 3:

**Table 3**

| | CH Mixture as of the invention | 50 g of Glucose | |
|---|---|---|---|
| | AUC (Mean $\pm$ SEM) | AUC (Mean $\pm$ SEM) | Value p[1] |
| Glucose 120 min (mmol/l) | 53 $\pm$ 14 | 62 $\pm$ 17 | 0,05 |
| Insulin 120 min | 1275 $\pm$ 323 | 3917 $\pm$ 603 | 0,001 |
| C-Peptide 120 min | 263 $\pm$ 23 | 605 $\pm$ 44 | 0,001 |
| Triglycerides 120 min | -1056 $\pm$ 287 | -1367$\pm$510 | 0,813 |
| [1]comparison of means: test T of Student. Significance level 95% | | | |

The following conclusions may be drawn, based on these findings:

- The glycemic index (GI) of the carbohydrate mixture as of the invention, taking 50 g of Glucose as standard, is the following:

$$GI(\%) = \frac{\text{AUC CH mixture of the invention}}{\text{AUC } s \tan dard} \times 100 = 85,48\%$$

- The glycemic load (GL) of the carbohydrate mixture as of the invention, taking 50g of glucose as the standard is **7.7 g/100 ml.**

- The calculation relating to the insulin response gives the following result:

**AUC Insulin = 32.50%**

- The area below the curve (AUC) relating to the C-Peptide gives the following result

**AUC C-Peptide = 43.50%**

As may be drawn from previous data, the carbohydrate mixture of the present invention successfully reduces the postprandial glycemic response, reducing the average glucose in blood concentrations and the secretion of insulin. Equally, owing to the fact it does not contain fructose, higher clearing of circulating lipids may be made. The carbohydrate mixture, therefore, as of the invention is useful for the nutritional treatment or for the prevention of diseases associated with the metabolic syndrome in general and diabetes in particular, primarily in type II diabetes. Equally, the carbohydrate mixture of the present invention increases the quantity of carbohydrates entering the colon and increases the fermentation and production of absorbable short chain fatty acids (SCFA), leading to the potential regulating of hepatic glyconeogenesis and insulin control, with obvious effects on the metabolism of the lipoproteins.

**Claims**

1. Mixture of carbohydrates for its use in a food product intended for enteral or oral nutrition **characterised in that** it contains type IV resistant starch, maltodextrin of low dextrose equivalent DE = 5-8, inulin and cellulose.

2. Mixture of carbohydrates according to claim 1, **characterised in that** it contains 50.98% in weight of type IV resistant starch, 33.74% in weight of maltodextrin 5-8 DE, 11.74% in weight of inulin and 3.54% in weight of cellulose, percentages respect to the total weight of the mixture.

3. Mixture of carbohydrates according to claim 1 or 2, **characterised in that** it is exempt from fructose.

4. Mixture of carbohydrates according to any of the previous claims **characterised in that** it also contains at least one additive selected from among sweeteners and stabilizers.

5. Mixture of carbohydrates according to claim 4, **characterised in that** the proportion of sweetener is that of 0.32% in weight with respect to the total weight of the mixture.

6. Mixture of carbohydrates according to claim 4, **characterised in that** the proportion of stabiliser is 0.22% in weight with respect to the total weight of the mixture.

7. Mixture of carbohydrates according to claim 1 or 2, **characterised in that** the maltodextrin of low glucose equivalent has a DE equal to 6.

8. Mixture of carbohydrates according to any of the previous claims **characterised in that** it contains sugars of 0.91% with respect to the total weight of the mixture.

9. Mixture of carbohydrates according to any of the previous claims **characterised in that** it has a glycemic index of 85.48% mmol/l.

10. Food product intended for enteral or oral nutrition **characterised in that** it includes a mixture of carbohydrates according to any of the previous claims.

11. Food product according to claim 10 for its use in the nutritional treatment or the prevention of diseases associated with the metabolic syndrome in general and diabetes in particular, primarily type II diabetes and its associated comorbidity.

**Patentansprüche**

1. Eine Mischung von Kohlenhydraten zur Verwendung in Nahrungsprodukten für die enterale oder orale Ernährung **dadurch gekennzeichnet, dass** sie resistente Stärke Typ IV, Maltodextrin mit einem niedrigen Dextroseaequivalent DE=5-8, Alantstärke und Zellulose enthält.

2. Eine Mischung von Kohlenhydraten gemäss Anspruch 1 **dadurch gekennzeichnet, dass** sie 50,98 Gew.% der resistenten Stärke Typ IV, 33,74 Gew.% Maltodextrin 5-8 DE, 11,74 Gew.% Alantstärke und 3,54 Gewi.% Zellulose enthält, wobei die Prozentangaben sich auf das Gesamtgewicht der Mischung beziehen.

3. Eine Mischung von Kohlenhydraten gemäss Anspruch 1 oder 2 **dadurch gekennzeichnet, dass** sie keinen Fruchtzucker enthält.

EP 2 294 932 B1

**4.** Eine Mischung von Kohlenhydraten gemäss einem der vorhergehenden Ansprüche **gekennzeichnet dadurch, dass** sie auch mindestens einen Zusatzstoff ausgewählt von Süssstoffen und Stabilisatoren enthält.

**5.** Eine Mischung von Kohlenhydraten gemäss Anspruch 4 **gekennzeichnet dadurch, dass** der Anteil des Süssstoffes 0,32 Gew.% bezogen auf das Gesamtgewicht der Mischung beträgt.

**6.** Eine Mischung von Kohlenhydraten gemäss Anspruch 4 **gekennzeichnet dadurch, dass** der Anteil des Stabilisators 0,22 Gew.% bezogen auf das Gesamtgewicht der Mischung beträgt.

**7.** Eine Mischung von Kohlenhydraten gemäss Anspruch 1 oder 2 **gekennzeichnet dadurch, dass** das niedrig glukoseaequivalente Maltodextrin eine DE gleich 6 aufweist.

**8.** Eine Mischung von Kohlenhydraten gemäss einem der vorhergehenden Ansprüche **gekennzeichnet dadurch, dass** sie 0,91% Zucker enthält bezogen auf das Gesamtgewicht der Mischung.

**9.** Eine Mischung von Kohlenhydraten gemäss einem der vorhergehenden Ansprüche **gekennzeichnet dadurch, dass** sie einen glykämischen Index von 85,48% mmol/l aufweist.

**10.** Ein Nahrungsmittel für enterale oder orale Ernährung **gekennzeichnet dadurch, dass** es eine Mischung von Kohlenhydraten gemäss einem der vorhergehenden Ansprüche enthält.

**11.** Ein Nahrungsmittel gemäss Ansprch 10 für den Gebrauch bei der Behandlung von Ernährungsproblemen oder zur Vorbeugung von Krankheiten im Zusammenhang mit Stoffwechselsyndromen im allgemeinen und besonders der Zuckerkrankheit, hauptsächlich der Zuckerkrankheit vom Typ II und der damit verbunden Begleiterkrankungen.

## Revendications

**1.** Mélange d'hydrates de carbone destiné à un produit alimentaire pour nutrition entérale ou orale, **caractérisé en ce qu'**il contient de l'amidon résistant de type IV, de la maltodextrine à faible équivalent en dextrose, avec un DE = 5-8, de l'inuline et de la cellulose.

**2.** Mélange d'hydrates de carbone selon la revendication 1, **caractérisé en ce qu'**il contient 50,98 % en poids d'amidon résistant de type IV ; 33,74 % en poids de maltodextrine, avec un DE de 5-8 ; 11,74 % en poids d'inuline ; et 3,54 % en poids de cellulose, des pourcentages en rapport avec le poids total du mélange.

**3.** Mélange d'hydrates de carbone selon la revendication 1 et 2, **caractérisé en ce qu'**il est sans fructose.

**4.** Mélange d'hydrates de carbone selon n'importe laquelle des précédentes revendications, **caractérisé en ce qu'**il contient également au moins un additif sélectionné parmi des édulcorants et agents stabilisants.

**5.** Mélange d'hydrates de carbone selon la revendication 4, **caractérisé en ce que** la proportion d'édulcorant est de 0,32 % en poids, par rapport au poids total du mélange.

**6.** Mélange d'hydrates de carbone selon la revendication 4, **caractérisé en ce que** la proportion d'agent stabilisant est de 0,22 % en poids, par rapport au poids total du mélange.

**7.** Mélange d'hydrates de carbone selon la revendication 1 ou 2, **caractérisé en ce que** la maltodextrine à faible équivalent en glucose possède un DE égal à 6.

**8.** Mélange d'hydrates de carbone selon n'importe laquelle des revendications précédentes, **caractérisé en ce qu'**il contient 0,91 % de sucres, par rapport au poids total du mélange.

**9.** Mélange d'hydrates de carbone selon n'importe laquelle des revendications précédentes, **caractérisé en ce qu'**il possède un indice glycémique de 85,48 % mmol/L.

**10.** Produit alimentaire destiné à la nutrition entérale ou orale, **caractérisé en ce qu'**il inclut un mélange d'hydrates de carbone selon n'importe laquelle des revendications précédentes.

**11.** Produit alimentaire selon la revendication 10 destiné à une utilisation dans le traitement nutritionnel ou la prévention des maladies associées au syndrome métabolique en général, et du diabète en particulier, principalement le diabète de type II et sa comorbidité associée.

**FIG. 1**

**FIG. 2**

**FIG. 3**

**FIG. 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004022074 A
- WO 2007004883 A

- ES 2211932 T3

**Non-patent literature cited in the description**

- *FAO Food and nutrition study,* 1997
- **CUMMINGS, J.H.** Carbohydrate: Terminology and Classification. *European Journal of Clinical Nutrition,* 2007
- **MANN, J.I.** Lines to legumes: changing concepts of diabetic diets. *Diabetic Medicine,* 1984, vol. 1, 191-198
- **BRAND, J.C. ; COLAGIURI, S. ; CROSSMAN, S. ; ALLEN, A. ROBERTS, D.C. ; TRUSWELL, A.S.** Low-glycemic index foods improve long-term glycemic control in NIDDM. *Diabetes Care,* 1991, vol. 14, 95-101

- *Consult FAO/WHO of experts on carbohydrates in human nutrition,* 1997
- *FAO/WHO Expert Consultation on Carbohydrates in Human Nutrition,* 1997
- **LÊ, K-A. ; TAPPY, L.** *Current opinion in Clinical Nutrition and Metabolic Care,* 2006, vol. 9, 469-475
- **JOHN P. BANTLE ; SUSAN K. RAATZ ; WILLIAM THOMAS ; ANGELIKI GEORGOPOULOS.** Effects of dietary fructose on plasma lipids in healthy subjects. *American Journal of Clinical Nutrition,* November 2000, vol. 72 (5), 1128-1134